# EUROPEAN PATENT APPLICATION

(11) **EP 1 422 240 A2**
(43) Date of publication of application: **26.05.2004**
(21) Application number: 03077844.3
(22) Date of filing: 10.09.2003
(51) Int. Cl.: C07K 14/665, A61K 38/33, A61P 25/04

(54) **Analogs of nociceptin**

(30) Priority: 24.09.2002 IT MI20022022
(71) Applicant: ITALFARMACO S.p.A., 20126 Milano (IT)
(72) Inventor: Guerrini, Remo, 44100 Ferrara (IT); Calo', Girolamo, 44100 Ferrara (IT); Salvadori, Severo, 44100 Ferrara (IT); Regoli, Domenico, 44100 Ferrara (IT)
(74) Representative: Pistolesi, Roberto

(57) **Abstract**

Peptide analogs of nociceptin, pharmaceutical composition containing thereof and their use in the treatment of disorders and/or phatological conditions where is useful a NOP receptors activation are described.

## Description

The present invention relates to peptide analogs of nocicettin able to modulate the ORL1 receptor (Opioid Receptor Like 1) activity, pharmaceutical compositions containing thereof and their use in the tretment of dysfunctions and/or pathological conditions where is involved the same receptor.

### Field of invention

A novel receptor structurally similar to opioid receptors has been cloned in 1994 and named "opioid receptor like 1" (ORL1 - according to the recent IUPHAR recommendations, the preferred name is NOP). The endogenous ligand of this receptor (nociceptin/orphanin FQ, N/OFQ), identified at the end of 1995, is an heptadecapeptide which shows similarity with some opioid peptides (e.g. Dynorphin A), but does not bind to classical opioid receptors of the mu (MOP), delta (DOP) or kappa (KOP) type. The cellular effects mediated by the NOP receptor are similar to those evoked by classical opioid receptors. From a structural and transductional point of view, the new peptide/receptor system N/OFQ-NOP belong to the opioid family, however it is pharmacologically distinct. During the 1996-98 period, several studies demonstrated that N/OFQ may modulate different functions both in the central nervous system (pain, anxiety, learning and memory, drug abuse, food intake) and in the periphery (blood pressure and hearth rate, renal, gastrointestinal, genitourinary and respiratory functions) (see for details Massi *et al.,* Peptides 21, 2000).

Since 1996 the present inventors have performed researches on the N/OFQ-NOP system that led to the identification of some original ligands for the NOP receptor such as a) N/OFQ(1-13)NH₂, which represents the minimum functional fragment as active as the natural ligand N/OFQ (Calo *et al., Eur J Pharmacol* **311**, R3-5, 1996); b) N/OFQ-NH₂ that, especially in vivo, produces more intense and prolonged effects than N/OFQ (Rizzi *et al., Naunyn Schmiedebergs Arch Pharmacol* **363**, 161-165. 2001); c) [Tyr¹]N/OFQ(1-13)NH₂ a mixed agonist acting on NOP and on classical opioid receptors (Calo *et al*., *Can J Physiol* *Pharmacol* **75,** 713-8, 1997; Varani *et al*., *Naunyn Schmiedebergs Arch Pharmacol* **360**, 270-7, 1999); d) [Phel¹ Ψ(CH₂-NH)Gly²]N/OFQ(1-13)NH₂ a selective ligand for the NOP receptor that behaves as a pure antagonist, partial agonist, or even full agonist in a preparation dependent manner (Guerrini *et al., Br J Pharmacol* **123**, 163-5, 1998; Okawa *et al., Br J Pharmacol* **127,** 123-30, 1999). From the detailed analysis of [Phe¹Ψ(CH₂-NH)Gly²]N/OFQ(1-13)NH₂ pharmacological behaviour reported in Calo' *et al..* (*Peptides* **21**, 935-47, 2000), it results that this compound is actually a NOP partial agonist; e) [Nphe¹]N/OFQ(1-13)NH₂ the first pure and competitive antagonist selective for the NOP receptor (Calo *et al., Br J Pharmacol* **129**, 1183-93, 2000; Guerrini *et al., J Med Chem* **15**, 2805-13, 2000). These ligands actions have been characterized in a variety of in vitro and in vivo assays (see Calo *et al., Br J Pharmacol* **129**, 1261-83, 2000). More recently, the Phe⁴ residue has been replaced by pFPhe or pNO₂Phe obtaininig potent, NOP selective, agonists (Guerrini *et al., J Med Chem* **44**, 3956-64, 2001). Another interesting compound, [Arg¹⁴,Lys¹⁵]N/OFQ was identified as being an highly potent (17 fold potent than N/OFQ) and selective agonist at recombinant human NOP receptors expressed in HEK293 cells (Okada *et al., Biochem Biophys Res Commun* **278**, 493-8, 2000). The actions of this ligand were further characterized in vitro in N/OFQ sensitive isolated tissues and in vivo in the mouse (Rizzi *et al., J Pharmacol Exp Ther* **300**, 57-63, 2002).

N/OFQ analogs have been disclosed in WO 99/07212, WO 97/07208, WO 99/03491 and WO 99/03880. These peptides are said to be useful for treating/preventing diseases related to: hyperalgesia, neuroendocrine functions, stress, locomotor activity and anxiety.

The reference sequence for the nociceptin peptide is the following:

H-Phe-Gly-Gly-Phe-Thr-Gly-Ala-Arg-Lys-Ser-Ala-Arg-Lys-Leu-Ala-Asn-Gln-
OH

### Description

The present invention concerns peptides analogs of nociceptin, having the following general formula (I) 
Phe¹-Ψ-Gly²-Gly³-Xaa⁴-Thr⁵-Gly⁶-Ala⁷-Arg⁸-Lys⁹-Ser¹⁰-Ala¹¹-Arg¹²-Lys¹³-Xbb¹⁴-Xcc¹⁵-
Asn¹⁶-Gln¹⁷-R
(I)
 wherein
Ψ represents the bond between the first two aminoacidic residues and is selected from CO-NH and CH₂-NH; **Xaa**^{**4**} is pXPhe wherein "X" represents H,Cl, Br, I, F, NO₂, CN and "p" indicates the para- position of the phenyl ring of Phe, Tic, Phg, Atc, Aic, which represent tetrahydroisochinolin-3-carboxylic acid, phenylglicin, aminotetralincarboxylic acid, aminoindancarboxylic acid, respectively; **Xbb**^{**14**} is Trp, Arg, Lys, Leu, Orn, homoArg, diaminobutyric acid, diaminopropionic acid; **Xcc**^{**15**} is Phe, Arg, Lys, Ala, Orn or Trp; R represents a terminal amide group (-NH₂) or a terminal carboxy group (-OH); wherein the aminoacidic residues or derivatives thereof can be in D or, preferably, L configuration.

The present invention also describes, the pharmaceutically acceptable salts of the compounds (I), in particular salts of organic acids and mineral acids, such as chlorhydrates, bromhydrates, phosphates, sulphates, acetates, ascorbates, tartrates, gluconates, benzoates, maleates, fumarates and stearates.

The compounds (I) in which Ψ is CO-NH or CH₂-NH; **Xaa**^{**4**} is pXPhe where "pX" is as above defined; **Xbb**^{**14**} is Arg, Lys, Leu or Orn; **Xcc**^{**15**} is Arg, Lys, Ala, Orn or Trp; **R** is -NH₂ or -OH are preferred.

The compounds (I) in which Ψ is CO-NH or CH₂-NH; **Xaa**^{**4**} is L-pFPhe or L-pNO_{2;} **Xbb**^{**14**} is L-Arg, L-Lys or L-Leu; **Xcc**^{**15**} is L-Arg, L-Lys or L-Ala; **R** is -NH₂ are most preferred.

The analogs of formula (I) in which the variable residues have the meanings reported in following table (Table 1) are even more preferred:

| | Ψ | Xaa | Xbb | Xcc | R |
|---|---|---|---|---|---|
| 1 | CO-NH | (pF)Phe | Arg | Lys | NH₂ |
| 2 | CH₂-NH | (pF)Phe | Arg | Lys | NH₂ |
| 3 | CO-NH | (pF)Phe | Arg | Arg | NH₂ |
| 4 | CH₂-NH | (pF)Phe | Arg | Arg | NH₂ |
| 5 | CO-NH | (pF)Phe | Lys | Lys | NH₂ |
| 6 | CH₂-NH | (pF)Phe | Lys | Lys | NH₂ |
| 7 | CO-NH | (pF)D-Phe | Arg | Lys | NH₂ |
| 8 | CH₂-NH | (pF)D-Phe | Arg | Lys | NH₂ |
| 9 | CO-NH | (pF)D-Phe | Arg | Arg | NH₂ |
| 10 | CH₂-NH | (pF)D-Phe | Arg | Arg | NH₂ |
| 11 | CO-NH | (pF)Phe | Arg | Ala | NH₂ |
| 12 | CH₂-NH | (pF)Phe | Arg | Ala | NH₂ |
| 13 | CO-NH | (pF)D-Phe | Leu | Lys | NH₂ |
| 14 | CH₂-NH | (pF)D-Phe | Leu | Lys | NH₂ |
| 15 | CO-NH | (pF)Phe | D-Arg | Lys | NH₂ |
| 16 | CH₂-NH | (pF)Phe | D-Arg | Lys | NH₂ |
| 17 | CO-NH | (pNO₂)Phe | Arg | Lys | NH₂ |
| 18 | CH₂-NH | (pNO₂)Phe | Arg | Lys | NH₂ |
| 19 | CO-NH | (pNO₂)Phe | Lys | Lys | NH₂ |
| 20 | CH₂-NH | (pNO₂)Phe | Lys | Lys | NH₂ |
| 21 | CO-NH | (pNO₂)Phe | Arg | Arg | NH₂ |
| 22 | CH₂-NH | (pNO₂)Phe | Arg | Arg | NH₂ |
| 23 | CO-NH | (pNO₂)Phe | Lys | D-Lys | NH₂ |
| 24 | CH₂-NH | (pNO₂)Phe | Lys | D-Lys | NH₂ |
| 25 | CO-NH | (pNO₂)D-Phe | Arg | Lys | NH₂ |
| 26 | CH₂-NH | (pNO₂)D-Phe | Arg | Lys | NH₂ |
| 27 | CO-NH | (pNO₂)D-Phe | Arg | Arg | NH₂ |
| 28 | CH₂-NH | (pNO₂)D-Phe | Arg | Arg | NH₂ |
| 29 | CO-NH | (pNO₂)Phe | Arg | Ala | NH₂ |
| 30 | CH₂-NH | (pNO₂)Phe | Arg | Ala | NH₂ |
| 31 | CO-NH | (pNO₂)Phe | Leu | Lys | NH₂ |
| 32 | CH₂-NH | (pNO₂)Phe | Leu | Lys | NH₂ |
| 33 | CO-NH | (pNO₂)Phe | D-Arg | D-Lys | NH₂ |
| 34 | CH₂-NH | (pNO₂)Phe | D-Arg | D-Lys | NH₂ |
| 35 | CO-NH | (pNO₂)Phe | Leu | D-Lys | NH₂ |
| 36 | CH₂-NH | (pNO₂)Phe | Leu | D-Lys | NH₂ |
| 37 | CO-NH | (pF)Phe | Arg | Lys | OH |
| 38 | CH₂-NH | (pF)Phe | Arg | Lys | OH |
| 39 | CO-NH | (pNO₂)Phe | Arg | Lys | OH |
| 40 | CH₂-NH | (pNO₂)Phe | Arg | Lys | OH |
| 41 | CO-NH | (pNO₂)Phe | Lys | Lys | OH |
| 42 | CH₂-NH | (pNO₂)Phe | Lys | Lys | OH |
| 43 | CO-NH | (pF)Phe | Arg | Arg | OH |
| 44 | CH₂-NH | (pF)Phe | Arg | Arg | OH |

Among these, the compounds in which Ψ is CO-NH or CH₂-NH; **Xaa**^{**4**} is L-pFPhe; **Xbb**^{**14**} is L-Arg; **Xcc**^{**15**} is L-Lys; **R** is -NH₂, represented by the following formulae, are most preferred : 
a)	H-Phe-Gly-Gly-(pF)Phe-Thr-Gly-Ala-Arg-Lys-Ser-Ala-Arg-Lys-Arg-
	Lys-Asn-Gln-NH2
 
b)	H-Phe-Ψ(CH2NH)-Gly-Gly-(pF)Phe-Thr-Gly-Ala-Arg-Lys-Ser-Ala-
Arg-Lys-Arg-Lys-Asn-Gln-NH2
 The peptide analogs of the present invention can be synthetized by different known techniques, as for instance Schroeder et al. " The Peptides" vol 1, Academic Press, 1965; Bodanszky et al. "Peptide Synthesis" Interscience Publisher, 1966; Baranay & Merrifield, " The peptides; Analysis, Synthesis, Biology", 2, Academic Press, 1980; E. Atheron e R. C. Sheppard, "Solid Phase Peptide Synthesis" IRL Press at Oxford University Press 1989; J. Jones, " The Chemical Synthesis of Pepdites", Claredon Press, Oxford 1994.

These tecniques comprise peptide synthesis in solid phase or in solution, synthetic methods of organic chemistry or any combination thereof. The selected synthesis scheme will obviously depend on the composition of the particular peptide. In preference, synthetic methods based on suitable combinations of techniques in solid phase and classic methods in solution, with low production costs, particularly on industrial scale, are used.

In details these methods consist in:
i) Solution synthesis of fragments of the peptide chain through the consecutive coupling of N-protected aminoacids, suitably activated, to an aminoacid or to a C-protected peptide chain, with isolation of the intermediates, subsequent selective deprotection of the N and C-terminus of these fragments and repeated coupling thereof until the peptide is obtained. Where is necessary the lateral chains are deprotected.
ii) Solid phase synthesis of the peptide chain from the C-terminus to the N-terminus on an insoluble polymeric support. The peptide is removed from the resin by hydrolysis with anhydrous hydrofluoric acid or trifluoracetic acid, with concomitant deprotection of the lateral chains.

At the end of the synthesis the peptides can be purified and isolated by treatment with convenient solvents and by chromatographic methods, as HPLC.

The peptide analogs of the present invention act as agonists of the NOP receptor.

The intrinsic activity of each compound may vary on the basis of the different kind of residue which is present in the sequence variable sections. For instance, a full agonist activity for the compound a) and a partial agonist activity for the compound b) was observed. However, the whole class of the compounds (I) showed a greater potency and a prolonged activation than the nociceptin peptide.

On the whole, the results of the in vitro and in vivo tests show the biological and pharmacological efficacy of the peptides of the present invention.

In a second aspect, the invention is directed to pharmaceutical compositions containing the peptide analogs herein described, if necessary in combination with pharmaceutically acceptable carriers and excipients.

The compositions of the present invention can be admnistered orally or parenterally, for instance respiratory, rectal, spinal, intrathecal and topical, in injectable preparation, capsule, tablet, granulate, solution, suspension, syrup, suppository, nasal spray, cream, ointment, gel, controlled release or other. The principles and methods for the preparation of fharmaceutical compositions are known and described, for instance, in Remington's Pharmaceutical Sciences, 18°Edition, Mack Publishing Company, Easton, Pa, 1990. The pharmaceutical compositions will contain an efficacy amount of peptides (or derivatives thereof) generally comprised between 0,1 and 1000 mg, preferably between 0,1 and 500 mg. The daily dosage will be different in accordance with the pathology/disfunction to be treated, with the form and administration route, age, sex, weight of the subject, with the state of health and with other parameters to be evalutated time to time, but normally may be comprised between 0,1 and 1000 µg/Kg of the body weight.

In consideration of the activity profile that the peptides of the invention showed in the in vitro and in vivo tests, the pharmaceutical compositions containing the described peptides can be used for the treatment of disfunctions, conditions or pathological states in which it is desirable to obtain a potent and prolonged activation of the NOP receptors, as hypertension, tachycardia, water-retaining diseases, heart failure, dysfunctions of the smooth muscle in the gastrointestinal, respiratory and genitourinary tracts (especially urinary incontinence due to neurogenic bladder), inflammatory disorders and peripheral and spinal analgesia, in particular in the tretment of the chronic pain or also in the inhibition of cough.

The high molecular weigh of the compounds and the presence of residues which may be positively charged at physiologically pH, makes the possibility to cross the brain blood barrier unlike. Even if the biodistribution is mainly peripheral, such compounds may have central effetcs following to local administration, e.g. may induce analgesia after intrathecal or spinal administration.

### EXPERIMENTAL SECTION

### 1. Peptides preparation

### 1.1 General scheme of synthesis

The peptides of the present invention were prepared by solid phase synthesis using a Fmoc-PAL-PEG-PS-resin. The aminoacids protected as fuorenil-methyl-oxycarbonyl (Fmoc) were assembled with diisopropylcarbodiimide (DIPCDI) and 1-hydroxybenzotriazole (HOBT) as coupling agents. Piperidine 20% in DMF was used to remove Fmoc group and the protected peptide resin was treated with reagent K to obtain the crude peptide. Compounds containing a peptide bond modification between the first two aminoacidic residues (Phe¹[Ψ (CH₂NH)]Gly²) were obtained by condensing Boc-Phe-CHO on the protected peptide(2-17) linked to the resin in the last synthesis step, reducing the intermediate imine derivative in situ with NaBH₃CN.

The preparative HPLC system was performed using a Waters Delta Prep 4000 system with a Waters Prep LC 40-mm assembly C18 column (30x4 cm, 300 A, 15mm spherical particle size column) and with a gradient made from two solvents: A) acetonitrile 10% v/v in H₂O; B) acetonitrile 60% in H₂O both containing 0.1% of trilfuoroacetic acid (TFA) and a linear gradient from 0 to 50% of solvent B in 25 min.

The same gradient was used for analytical runs on Alltech C18 (4.6 x 150 mm 5-µm particle size) column over a period of 30 min using the following linear gradients: I) from 0 to 50% solvent B, II) from 0% to 20% solvent B and III) from 0% to 100% solvent B. All peptides showed less than 1% impurities when monitored at 220 nm.

Molecular weights of the compounds were determined by Maldi-Tof using a Helwett-Packard G2025 A LD-TOF system mass spectrometer. Optical rotation was determined using a Perkin-Elmer 241 polarimeter with a 10 cm cell using methanol at a peptide concentration of 1%. For the intermediates of some peptides a 1HNMR spectroscopy was performed with a 200 MHz Bruker Instrument.

### 1.2 Process

The peptide analogs reported in the Table were obtained with the process herebelow described in details for the syntesis of peptides a) and b).

Fmoc-PAL-PEG-PS resin, (0.18 mmol/g, 0.2 g) was treated with piperidine (20%) in DMF and linked with Fmoc-Gln(Trt)-OH, via its N-hydroxybenzotriazole active ester. The following Fmoc amino acids were sequentially coupled to the growing peptide chain: Fmoc-Asn(Trt)-OH, Fmoc-Lys(Boc)-OH, Fmoc-Arg(Pmc)-OH, Fmoc-Lys(Boc)-OH, Fmoc-Arg(Pmc)-OH, Fmoc-Ala-OH, Fmoc-Ser(tBu)-OH, Fmoc-Lys(Boc)-OH, Fmoc-Arg(Pmc)-OH, Fmoc-Ala-OH, Fmoc-Gly-OH, Fmoc-Thr(tBu)-OH, Fmoc-(pF)Phe-OH, Fmoc-Gly-OH, Fmoc-Gly-OH, Fmoc-Phe-OH. All the Fmoc amino acids (4 equiv) were coupled to the growing peptide chain by using 1,3-diisopropylcarbodiimide (4 equiv) and 1-hydroxybenzotriazole (4 equiv) in DMF, and the coupling reaction time was 1h. Piperidine (20%) in DMF was used to remove the Fmoc group at all steps. After deprotection of the last N^{α}-Fmoc group, the peptide resin was washed with methanol and dried in vacuo to yield the protected [(pF)Phe⁴, Arg¹⁴ , Lys¹⁵]-NC(1-17)-PAL-PEG-PS-Resin. This protected peptide-resin was treated with reagent K (TFA / H₂O / phenol / ethanedithiol / thioanisole 82.5 : 5 : 5: 2.5: 5; v/v; 10 mL / 0.2 g of resin) for 1 h at room temperature. After filtration of the exhausted resin, the solvent was concentrated in vacuo and the residue triturated with ether. The crude peptide was purified by preparative reverse phase HPLC to yield a white powder after lyophilization.

The synthesis of [Phe¹Ψ (CO-NH) Gly², (pF)Phe⁴, Arg¹⁴, Lys¹⁵]-N/OFQ-NH₂ was performed starting from the intermediate [(pF)Phe⁴, Arg¹⁴, Lys¹⁵]-N/OFQ-(2-17)-resin synthetised as described above. This intermediate (0.2 g, 0.21 mmol/g, 0.042 mmol) was swelled in methanol containig 1% (V/V) acetic acid (2 mL). After 20 min, a solution of Boc-Phe-CHO (0.016 g, 0.063 mmol) and NaBH3CN (0.008 g, 0.13 mmol) dissolved in methanol (0.4 mL) was added and the reaction mixture stirred for 1h. After this time, the resin was washed with methanol and treated with reagent K as reported above.

### 2. Pharmacological Tests

### 2.1 In vitro assays

The compounds of the Table have been evaluated in vitro in several N/OFQ-sensitive isolated tissues from various species such as the rat, mouse and guinea pig and in Chinese hamster ovary cells expressing the human recombinant NOP receptor (CHO_{hNOP}). The conditions adopted for studying the effects of the compounds in the electrically stimulated tissues (mouse and rat vas deferens, guinea pig ileum) are described in *Bigoni et al. (Naunyn Schmiedebergs Arch Pharmacol* **359**, 160-7, 1999, herein incorporated by reference), whereas the conditions adopted for investigating the effects in CHO_{hNOP} cells are disclosed in Okawa et al. *(Br J Pharmacol* **127**, 123-130, 1999, herein incorporated by reference). In each series of experiments the activity of the compounds was compared to that of the natural peptide N/OFQ.

### 2.2 In vivo assays

The peptides were evaluated in vivo for their ability to mimic or block a series of biological actions known to be mediated by the N/OFQ-NOP receptor system, such as: i) the pronociceptive and antimorphine effects induced by N/OFQ after intracerebroventricular (i.c.v.) application in mice (Calo *et al., Br J Pharmacol* **125**, 373-8, 1998), ii) the inhibition of spontaneous locomotor activity evoked by i.c.v. injection of N/OFQ in mice (Rizzi *et al., Naunyn Schmiedebergs Arch Pharmacol* **363**, 161-5, 2001). All these effects of N/OFQ were proven to be efficiently antagonized by the NOP selective antagonist [Nphe¹]N/OFQ(1-13)NH₂. In each series of experiments the actions of the new N/OFQ analogs were compared with those induced by the natural sequence N/OFQ.

### 2.3 Results

In isolated tissues the compounds having structure [Phe¹Ψ(CO-NH)Gly²] mimicked the effects of N/OFQ inducing similar maximal effects, thus acting as full agonists, while those with the structure [Phel¹Ψ(CH₂-NH)Gly²] behaved as partial agonists producing lower maximal effects than N/OFQ. Moreover, the potencies of all the compounds were very different ranging from potencies higher than that of N/OFQ (for instance [(pF)Phe⁴ , Arg¹⁴, Lys¹⁵]-NC-NH₂), similar to that of N/OFQ ([(pCl)Phe⁴]-N/OFQ-NH₂) or lower (([(pI)Phe⁴]-N/OFQ-NH₂). Those compounds which behaved as highly potent agonists or partial agonists (potencies higher than that of N/OFQ) displayed pEC₅₀ values 3 to 30 fold higher than N/OFQ depending on the preparation under study. The same compounds were evaluated for their ability to inhibit forskolin stimulated cAMP accumulation in CHO_{hNOP} cells. In this model all the compounds behaved as full agonists, their order of potency being the same as that found in isolated tissues. Those compounds which behaved as highly potent agonists displayed pEC₅₀ values > 3 fold higher than N/OFQ. The effect of those compounds acting as agonists are mediated by NOP receptor activation since they are unaffected by naloxone while blocked by [Nphe¹]N/OFQ(1-13)NH₂ which displayed similar pA₂ values (6.0 - 6.4) when tested vs these novel compounds or vs N/OFQ. Some of the compounds evaluated in vitro were also tested in a battery of in vivo assays of biological functions regulated by N/OFQ. In all the assays most of the analogs mimicked the effects evoked by the natural peptide. In the locomotor activity assay some compounds have been tested at 1 nmol i.c.v. dose. These mimicked the inhibitory effect of N/OFQ, however while the effects of the natural peptide lasted for about 20 min, those induced by some of the novel derivatives were still evident after several hours. Some analogs like [(pF)Phe⁴,Arg¹⁴,Lys¹⁵]-N/OFQ-NH₂ were found to be more than 10 fold more potent than N/OFQ. Similar results were also obtained in the mouse tail withdrawal assay were the compounds mimicked the hyperalgesic actions of N/OFQ.

As an example, the following table summarizes the results obtained in vitro and in vivo with the compound [(pF)Phe⁴,Arg¹⁴,Lys¹⁵]-N/OFQ-NH₂ (UFP-102) in comparison with the reference peptide N/OFQ.

| ***in vitro studies*** | | | | | |
|---|---|---|---|---|---|
| | | N/OFQ | | UFP-102 | |
| Preparation | Effect | pEC₅₀ | Eₘₐₓ | pEC₅₀ | Eₘₐₓ |
| CHO_{hNOP} | Stimulation binding. GTP S | 8.70 | 10.71 ± 1.50 | 10.12 | 12.26 ± 1.37 |
| CHO_{hNOP} | cAMP inhibition | 9.86 | 103 ±4% | 10.17 | 104 ± 3% |
| mVD | Electrically induced twitch inhibition | 7.77 | 91 ± 2% | 9.36 | 94 ± 1% |
| gpI | Electrically induced twitch inhibition | 8.04 | 56 ±5% | 8.98 | 55 ± 4% |

| ***in vivo studies*** | | | | | |
|---|---|---|---|---|---|
| | | N/OFQ | | UFP-102 | |
| Assay | Effect | Effective dose (nmol) | Duration of action | Effective dose (nmol) | Duration of action |
| Locomotor activity | depression | 1 | 20 min | 0.1 | >3 h |
| Tail withdrawal | pronociception | 1 | 15 min | 0.1 | > 2 h |

## Claims

1. Peptide analogs of nociceptin having general formula (I) : 
Phe¹-**Ψ**-Gly²-Gly³-**Xaa**⁴-Thr⁵-Gly⁶-Ala⁷-Arg⁸-Lys⁹-Ser¹⁰-Ala¹¹-Arg¹²-Lys¹³-**Xbb**¹⁴-**Xcc**¹⁵-
Asn¹⁶-Gln¹⁷-**R**
(I)
 wherein
Ψ represents the bond between the first two aminoacidic residues and is selected from CO-NH and CH₂-NH; **Xaa**^{**4**} is selected from pXPhe where "X" represents H,Cl, Br, I, F, NO₂, CN and "p" indicates the para- position of the phenyl ring of Phe, Tic, Phg, Atc, Aic, which represent tetrahydroisochinolin-3-carboxylic acid, phenylglicin, aminotetralincarboxylic acid, aminoindancarboxylic acid, respectively; **Xbb**^{**14**} is selected from Trp, Arg, Lys, Leu, Orn, homoArg, diaminobutyric acid, diaminopropionic acid; **Xcc**^{**15**} is selected from Phe, Arg, Lys, Ala, Om or Trp; **R** represents a terminal amide group (-NH₂) or a terminal carboxy group (-OH); wherein the aminoacidic residues or derivatives thereof can be in D or L configuration; and pharmaceutically acceptable salts thereof.

2. Peptide according to Claim 1, wherein Ψ is CO-NH or CH₂-NH; **Xaa**^{**4**} is Phe, pClPhe, pBrPhe, pIPhe, PNO₂Phe or pCNPhe; **Xbb**^{**14**} is Arg, Lys, Leu, Orn; **Xcc**^{**15**} is Arg, -Lys, Ala, Orn or Trp, **R** is -NH₂ or -OH.

3. Peptide according to Claim 2, wherein Ψ is CO-NH or CH₂-NH; **Xaa**^{**4**} is L-pFPhe or L-PNO₂Phe; **Xbb**^{**14**} is L-Arg, L-Lys, or L-Leu; **Xcc**^{**15**} is L-Arg, L-Lys or L-Ala; **R** is -NH₂.

4. Peptide according to Claim 3, selected from the group comprising:
Ψ is CO-NH; Xaa⁴ is Phe(pF); Xbb¹⁴ is Arg; Xcc¹⁵ is Lys; R is NH₂
Ψ is CH₂-NH; Xaa⁴ is Phe(pF); Xbb¹⁴ is Arg; Xcc¹⁵ is Lys; R is NH₂
Ψ is CO-NH; Xaa⁴ is Phe(pF); Xbb¹⁴ is Arg; Xcc¹⁵ is Lys; R is OH
Ψ is CH₂-NH; Xaa⁴ is Phe(pF); Xbb¹⁴ is Arg; Xcc¹⁵ is Lys; R is OH
Ψ is CO-NH; Xaa⁴ is Phe(pF); Xbb¹⁴ is Arg; Xcc¹⁵ is Arg; R is NH₂
Ψ is CH₂-NH; Xaa⁴ is Phe(pF); Xbb¹⁴ is Arg; Xcc¹⁵ is Arg; R is NH₂
Ψ is CO-NH; Xaa⁴ is Phe(pF); Xbb¹⁴ is Arg; Xcc¹⁵ is Arg; R is OH
Ψ is CH₂-NH; Xaa⁴ is Phe(pF); Xbb¹⁴ is Arg; Xcc¹⁵ is Arg; R is OH
Ψ is CO-NH; Xaa⁴ is Phe(pF); Xbb¹⁴ is Arg; Xcc¹⁵ is D-Lys; R is NH₂
Ψ is CH₂-NH; Xaa⁴ is Phe(pF); Xbb¹⁴ is Arg; Xcc¹⁵ is D-Lys; R is NH₂
Ψ is CO-NH; Xaa⁴ is Phe(pF); Xbb¹⁴ is Arg; Xcc¹⁵ is D-Lys; R is OH
Ψ is CH₂-NH; Xaa⁴ is Phe(pF); Xbb¹⁴ is Arg; Xcc¹⁵ is D-Lys; R is OH
Ψ is CO-NH; Xaa⁴ is Phe(pF); Xbb¹⁴ is D-Arg; Xcc¹⁵ is Lys; R is NH₂
Ψ is CH₂-NH; Xaa⁴ is Phe(pF); Xbb¹⁴ is D-Arg; Xcc¹⁵ is Lys; R is NH₂
Ψ is CO-NH; Xaa⁴ is Phe(pF); Xbb¹⁴ is D-Arg; Xcc¹⁵ is Lys; R is OH
Ψ is CH₂-NH; Xaa⁴ is Phe(pF); Xbb¹⁴ is D-Arg; Xcc¹⁵ is Lys; R is OH
Ψ is CO-NH; Xaa⁴ is Phe(pF); Xbb¹⁴ is D-Arg; Xcc¹⁵ is D-Lys; R is NH₂
Ψ is CH₂-NH; Xaa⁴ is Phe(pF); Xbb¹⁴ is D-Arg; Xcc¹⁵ is D-Lys; R is NH₂
Ψ is CO-NH; Xaa⁴ is Phe(pF); Xbb¹⁴ is D-Arg; Xcc¹⁵ is D-Lys; R is OH
Ψ is CH₂-NH; Xaa⁴ is Phe(pF); Xbb¹⁴ is D-Arg; Xcc¹⁵ is D-Lys; R is OH
Ψ is CO-NH; Xaa⁴ is D-Phe(pF); Xbb¹⁴ is Arg; Xcc¹⁵ is Lys; R is NH₂
Ψ is CH₂-NH; Xaa⁴ is D-Phe(pF); Xbb¹⁴ is Arg; Xcc¹⁵ is Lys; R is NH₂
Ψ is CO-NH; Xaa⁴ is D-Phe(pF); Xbb¹⁴ is Arg; Xcc¹⁵ is Lys; R is OH
Ψ is CH₂-NH; Xaa⁴ is D-Phe(pF); Xbb¹⁴ is Arg; Xcc¹⁵ is Lys; R is OH
Ψ is CO-NH; Xaa⁴ is Phe(pNO₂); Xbb¹⁴ is Arg; Xcc¹⁵ is Lys; R is NH₂
Ψ is CH₂-NH; Xaa⁴ is Phe(pNO₂); Xbb¹⁴ is Arg; Xcc¹⁵ is Lys; R is NH₂
Ψ is CO-NH; Xaa⁴ is Phe(pNO₂); Xbb¹⁴ is Arg; Xcc¹⁵ is Lys; R is OH
Ψ is CH₂-NH; Xaa⁴ is Phe(pNO₂); Xbb¹⁴ is Arg; Xcc¹⁵ is Lys; R is OH
Ψ is CO-NH; Xaa⁴ is Phe(pNO₂); Xbb¹⁴ is Arg; Xcc¹⁵ is Arg; R is NH₂
Ψ is CH₂-NH; Xaa⁴ is Phe(pNO₂); Xbb¹⁴ is Arg; Xcc¹⁵ is Arg; R is NH₂
Ψ is CO-NH; Xaa⁴ is Phe(pNO₂); Xbb¹⁴ is Arg; Xcc¹⁵ is Arg; R is OH
Ψ is CH₂-NH; Xaa⁴ is Phe(pNO₂); Xbb¹⁴ is Arg; Xcc¹⁵ is Arg; R is OH
Ψ is CO-NH; Xaa⁴ is Phe(pNO₂); Xbb¹⁴ is Arg; Xcc¹⁵ is D-Lys; R is NH₂
Ψ is CH₂-NH; Xaa⁴ is Phe(pNO₂); Xbb¹⁴ is Arg; Xcc¹⁵ is D-Lys; R is NH₂
Ψ is CO-NH; Xaa⁴ is Phe(pNO₂); Xbb¹⁴ is Arg; Xcc¹⁵ is D-Lys; R is OH
Ψ is CH₂-NH; Xaa⁴ is Phe(pNO₂); Xbb¹⁴ is Arg; Xcc¹⁵ is D-Lys; R is OH
Ψ is CO-NH; Xaa⁴ is Phe(pNO₂); Xbb¹⁴ is D-Arg; Xcc¹⁵ is Lys; R is NH₂
Ψ is CH₂-NH; Xaa⁴ is Phe(pNO₂); Xbb¹⁴ is D-Arg; Xcc¹⁵ is Lys; R is NH₂
Ψ is CO-NH; Xaa⁴ is Phe(pNO₂); Xbb¹⁴ is D-Arg; Xcc¹⁵ is Lys; R is OH
Ψ is CH₂-NH; Xaa⁴ is Phe(pNO₂); Xbb¹⁴ is D-Arg; Xcc¹⁵ is Lys; R is OH
Ψ is CO-NH; Xaa⁴ is Phe(pNO₂); Xbb¹⁴ is D-Arg; Xcc¹⁵ is D-Lys; R is NH₂
Ψ is CH₂-NH; Xaa⁴ is Phe(pNO₂); Xbb¹⁴ is D-Arg; Xcc¹⁵ is D-Lys; R is NH₂
Ψ is CO-NH; Xaa⁴ is Phe(pNO₂); Xbb¹⁴ is D-Arg; Xcc¹⁵ is D-Lys; R is OH
Ψ is CH₂-NH; Xaa⁴ is Phe(pNO₂); Xbb¹⁴ is D-Arg; Xcc¹⁵ is D-Lys; R is OH
Ψ is CO-NH; Xaa⁴ is D-Phe(pNO₂); Xbb¹⁴ is Arg; Xcc¹⁵ is Lys; R is NH₂
Ψ is CH₂-NH; Xaa⁴ is D-Phe(pNO₂); Xbb¹⁴ is Arg; Xcc¹⁵ is Lys; R is NH₂
Ψ is CO-NH; Xaa⁴ is D-Phe(pNO₂); Xbb¹⁴ is Arg; Xcc¹⁵ is Lys; R is OH
Ψ is CH₂-NH; Xaa⁴ is D-Phe(pNO₂); Xbb¹⁴ is Arg; Xcc¹⁵ is Lys; R is OH

5. Peptide according to Claim 4, selected from the group comprising :

a)H-Phe-Gly-Gly-(pF)Phe-Thr-Gly-Ala-Arg-Lys-Ser-Ala-Arg-Lys-
Arg-Lys-Asn-Gln-NH2

b)H-Phe-Ψ(CH2NH)-Gly-Gly-(pF)Phe-Thr-Gly-Ala-Arg-Lys-Ser-Ala-
Arg- Lys-Arg-Lys-Asn-Gln-NH2

6. Peptide according to Claims 1 to 5, for use as a medicament.

7. Pharmaceutical composition containing a peptide accordin to Claims 1 to 5, together with pharmaceutically accettable excipients.

8. Use of a peptide according to Claims 1 to 5, for the preparation of a medicament for the treatment of disorder conditions or pathological states in which it is desirable to obtain the activation of the NOP receptors.

9. Use according to Claim 8, in the treatment of hypertension, tachycardia, water-retaining diseases, hyponatremia, heart failure, motility dysfunctions of the smooth muscle in the gastrointestinal, respiratory and genitourinary tracts, inflammatory states, peripheral and spinal analgesia, inhibition of cough.

10. Use according to Claim 8, for the preparation of an analgesic.

11. Use according to Claim 8, for the preparation of a medicament useful in the treatment of urinary incontinence due to neurogenic bladder and bladder hyperactivity.

12. Use according to Claim 8, for the preparation of a medicament useful in the treatment of cough.

13. Use according to Claim 8, for the preparation of a medicament useful in the treatment of gastrointestinal hypermotility.

14. Use according to Claim 8, for the preparation of a medicament useful in the treatment of hyponatriemiam heart failure and hypertension.

15. Use according to Claim 8, for the preparation of a medicament useful in the treatment of chronic pain.
